# FASCICULE DE BREVET EUROPEEN

(11) **EP 2 131 915 B1**
(45) Date de publication et mention de la délivrance du brevet: **29.04.2015**
(21) Numéro de dépôt: 08775716.7
(22) Date de dépôt: 12.03.2008
(51) Int. Cl.: A61M 35/00, A61B 17/20, A45D 34/04

(54) **DISPOSITIF POUR L'APPLICATION CUTANEE DE SUBSTANCES**
VORRICHTUNG ZUR ANWENDUNG VON SUBSTANZEN AUF DER HAUT
DEVICE FOR THE CUTANEOUS APPLICATION OF SUBSTANCES

(30) Priorité: 13.03.2007 FR 0753787
(43) Date de publication de la demande: 16.12.2009
(73) Titulaire: DBV Technologies, 92220 Bagneux (FR)
(72) Inventeur: BIRY, Jean-François, F-75018 Paris (FR); EHOUARN, Pascale, F-91190 Gif Sur Yvette (FR); DONNE, Nathalie, F-75012 Paris (FR); DUPONT, Bertrand, F-13100 Aix En Provence (FR)
(74) Mandataire: Becker, Philippe
(86) Numéro de dépôt international: PCT/FR2008/050419
(87) Numéro de publication internationale: WO 2008/132358

(56) Documents cités:
- WO-A-2004/052425
- FR-A- 2 527 450
- US-A- 4 781 705
- US-A1- 2004 189 470

## Description

La présente invention concerne de façon générale les dispositifs pour l'application épi- ou trans- cutanée de substances biologiquement actives. L'invention concerne plus particulièrement les patchs ou timbres transdermiques destinés à faciliter l'absorption cutanée de telles substances, notamment à des fins de vaccination, désensibilisation ou d'administration de médicaments.

L'épiderme humain constitue une barrière contre l'entrée dans le corps d'agents extérieurs. La peau n'est pas étanche, elle est en fait perméable à un très grand nombre de substances avec un degré de perméabilité variable.

L'absorption percutanée correspond au transfert d'une substance à travers la peau depuis le milieu extérieur jusqu'au sang. Cette absorption est définie comme la somme de deux phénomènes : une pénétration des molécules au sein de la peau entière, suivie d'une résorption par la circulation sanguine ou lymphatique depuis le derme papillaire puis le derme profond. L'étape de pénétration est physiquement une diffusion passive à travers chaque structure du tégument : la couche cornée, l'épiderme de Malpighi, le derme et les annexes cutanées. Une fois absorbée, la substance est distribuée dans l'organisme puis, après avoir été ou non métabolisée, elle est éliminée. Les étapes succédant à l'absorption percutanée sont similaires à celles qui sont rencontrées pour toute autre voie de contamination.

La couche cornée constitue la barrière la plus efficace contre la pénétration d'une substance ; anatomiquement, la substance peut pénétrer par deux voies : l'une à travers les espaces intercellulaires de la couche cornée et à travers les cellules cornées elles-mêmes, l'autre par l'intermédiaire des annexes cutanées. Dans le cas d'un vaccin, une fois la couche cornée traversée, la substance est à même de rencontrer les cellules immunologiquement compétentes, et, en particulier, les cellules présentatrices d'antigènes comme les cellules de Langerhans dont le rôle est primordial dans la réaction immunologique de l'organisme. Faciliter le passage initial de l'antigène à travers la couche cornée, c'est rendre la vaccination épicutanée plus efficace. Dans le cas d'un patch destiné à l'administration d'une molécule pour un traitement systémique, diminuer temporairement l'efficacité de la couche cornée en tant que barrière, permet d'ouvrir la voie à l'administration transcutanée de principes actifs à forts poids moléculaires et, d'une façon générale, d'améliorer la vitesse d'administration des principes actifs.

L'absorption percutanée d'une substance au moyen d'un patch présente d'importants avantages par rapport à une injection par aiguille : absence de risque de contamination par atteinte de la peau, absence de douleur, facilité de transport, conservation rallongée de la substance active ou encore administration de la substance par le patient lui-même.

Un tel procédé d'administration présente cependant aussi des inconvénients. En effet, à la différence d'une injection sous-cutanée, une durée minimale d'application est nécessaire pour aboutir à l'effet de la substance du patch. Ainsi, une vaccination ou une immunisation peuvent être incomplètes si le patch n'a pas été appliqué suffisamment longtemps sur la peau. La durée d'application nécessaire étant très supérieure à la durée d'une injection, un praticien ne peut pas rester à proximité du patient pendant cette durée. Le praticien n'a alors pas la certitude que le patient ait maintenu le patch en contact avec la peau pendant une durée suffisante.

Le brevet FR 2,527,450 porte sur un dispositif de libération d'une substance dans la peau composé par exemple d'un bracelet en élastomère chargé en substance active. Toutefois, le dispositif proposé est réutilisable et ne permet donc pas d'empêcher le retrait prématuré du patch, ni de s'assurer qu'un temps de contact suffisant a été respecté par le patient.

L'invention vise à résoudre un ou plusieurs de ces inconvénients. L'invention a ainsi pour objet un dispositif pour l'application cutanée d'une substance, comprenant :
- un patch comprenant ladite substance; et
- un bracelet à usage unique comprenant un système de fixation irréversible permettant le maintien de la substance du patch contre la peau d'un sujet.

L'invention propose ainsi un dispositif pour l'application cutanée d'une substance permettant, par un système de fixation irréversible à usage unique, d'empêcher le retrait prématuré du dispositif par le patient et/ou dé s'assurer qu'un temps de contact suffisant avec la peau a été respecté.

Selon une variante, le bracelet comprend deux parties de fixation et le système de fixation irréversible du bracelet comprend des moyens pour fixer ensemble de façon irréversible les deux parties de fixation, le bracelet formant une boucle lorsque les deux parties de fixation sont jointes.

Selon une autre variante, les deux parties de fixation comprennent des surfaces mutuellement adhésives.

Selon encore une variante :
- une des deux parties de fixation comprend un organe de verrouillage présentant un alésage, et comprend un plot apte à être inséré de façon irréversible à travers l'alésage ;
- l'autre des deux parties de fixation comprend au moins un alésage susceptible d'être traversé par le plot.

Selon encore une autre variante, l'organe de verrouillage présente un anneau délimitant l'alésage et déformable élastiquement de façon à permettre l'insertion irréversible du plot.

Selon une variante, le dispositif comprend des moyens de solidarisation du patch au bracelet.

Selon encore une variante, les moyens de solidarisation du patch au bracelet sont irréversibles.

Selon une autre variante, les moyens de solidarisation irréversible comprennent une surface du patch ou du bracelet enduite d'adhésif et recouverte d'un élément pelable de protection.

Selon encore une variante, les moyens de solidarisation irréversible du patch au bracelet comprennent un organe du patch et un organe du bracelet mutuellement encliquetables.

Selon encore une autre variante, le dispositif comprend un conditionnement contenant le bracelet et le patch.

Selon une variante, le bracelet présente une surface en contact avec la substance.

Selon encore une variante, le bracelet présente une surface adhésive à proximité de la substance.

Selon une autre variante, la surface adhésive entoure la substance.

Selon encore une autre variante, le patch comprend un support muni d'une face présentant une surface en contact avec la substance et une surface adhésive destinée à entrer en contact avec la peau.

Selon une variante, le patch comprend un élément de protection pelable solidaire, le cas échéant, du support ou du bracelet et recouvrant la substance.

Selon encore une variante, le bracelet recouvre la substance.

Un autre objet de l'invention concerne l'utilisation d'un dispositif tel que défini ci-avant pour l'application d'une substance sur la peau, et/ou pour la délivrance d'une substance par voie épi- ou trans-cutanée à un sujet, notamment un mammifère, en particulier un être humain (par exemple enfant ou adulte). Le dispositif peut notamment être utilisé pour la vaccination de sujets, pour la désensibilisation de sujets, pour tester la sensibilité de sujets à des substances, ou pour la délivrance de toute substance active, telle que notamment des (poly)peptides biologiquement actifs et/ou antigéniques, par exemple.

Le dispositif peut ainsi être utilisé pour la vaccination de sujets contre tout pathogène. Ainsi, un objet particulier de l'invention réside dans une méthode de vaccination d'un sujet contre un pathogène, comprenant (i) l'application du dispositif selon l'invention sur la peau d'un sujet, la substance comprenant un antigène spécifique dudit pathogène, (ii) la fixation irréversible du bracelet autour d'un membre du sujet et le maintien du bracelet, de préférence au même endroit, pour une période de temps permettant le transfert de l'antigène dans la peau. Le pathogène peut être de nature variée (virus, bactérie, parasite, etc.) et l'antigène est typiquement de nature polypeptidique ou lipidique.

Le dispositif peut également être utilisé pour la désensibilisation de sujets à des allergènes. Ainsi, un objet particulier de l'invention réside dans une méthode de désensibilisation d'un sujet à un allergène, comprenant (i) l'application d'un dispositif selon l'invention sur la peau d'un sujet, la substance comprenant un antigène spécifique dudit allergène, (ii) la fixation irréversible du bracelet autour d'un membre du sujet et le maintien du bracelet, de préférence au même endroit, pour une période de temps permettant le transfert de l'antigène dans la peau.

Le dispositif peut également être utilisé pour la délivrance de toute substance active. Ainsi, un objet particulier de l'invention réside dans une méthode de délivrance d'une substance active à un sujet, comprenant (i) l'application d'un dispositif selon l'invention sur la peau d'un sujet, la substance comprenant un antigène spécifique dudit allergène, (ii) la fixation irréversible du bracelet autour d'un membre du sujet et le maintien du bracelet, de préférence au même endroit, pour une période de temps permettant le transfert de la substance dans la peau. La substance est typiquement de nature polypeptidique, comme une hormone, une cytokine, un facteur de croissance, un facteur trophique, etc.

La substance contenue dans le patch peut être formulée dans tout véhicule ou excipient adapté, et peut être sous forme solide (poudre), liquide (par exemple gel, pâte, solution), etc.

Un autre objet de l'invention concerne un procédé de fabrication d'un dispositif tel que défini ci-dessus, comprenant une étape d'assemblage fonctionnel entre un patch et un bracelet à usage unique et à fixation irréversible.

D'autres caractéristiques et avantages de l'invention ressortiront clairement de la description qui en est faite ci-après, à titre indicatif et nullement limitatif, en référence aux dessins annexés, dans lesquels :
- la figure 1 est une vue de dessous d'un premier mode de réalisation de dispositif d'application cutanée selon l'invention, prêt à être mis en place ;
- la figure 2 est une vue en coupe transversale du dispositif de la figure 1 ;
- la figure 3 est une vue en coupe transversale du bracelet après sa mise en place ;
- la figure 4 est une vue en coupe transversale d'un dispositif formé d'un kit, selon un deuxième mode de réalisation ;
- la figure 5 est une vue en coupe transversale d'un dispositif formé d'un kit selon un deuxième mode de réalisation.

Par la suite, les termes fixation irréversible ou inviolable définiront une fixation qui doit au moins partiellement être détruite pour être défaite.

L'invention propose un dispositif pour l'application cutanée d'une substance, permettant de déterminer ou de s'assurer de façon certaine qu'une substance a été appliquée suffisamment longtemps. Le dispositif comprend un patch incluant ladite substance destinée à pénétrer ou à entrer en contact avec l'épiderme, et un bracelet à usage unique muni d'un système de fixation irréversible permettant d'éviter le retrait prématuré du dispositif et/ou de s'assurer qu'un temps de contact suffisant de la substance contre la peau d'un sujet a été respecté.

Le patch peut être de toute nature, et il peut ainsi s'agir de tout dispositif ou de tout support ou surface appliqué sur la peau d'un sujet afin d'y appliquer une substance. Il peut s'agir d'un patch, occlusif ou non, d'un timbre, pansement, etc.. Le patch peut être composé de différents matériaux (polymère(s), pastique, métal, timbre, etc.), et la substance peut être liée au patch de différentes façons (imprégnation, liaison covalente, réservoir, forces électrostatique, etc.).

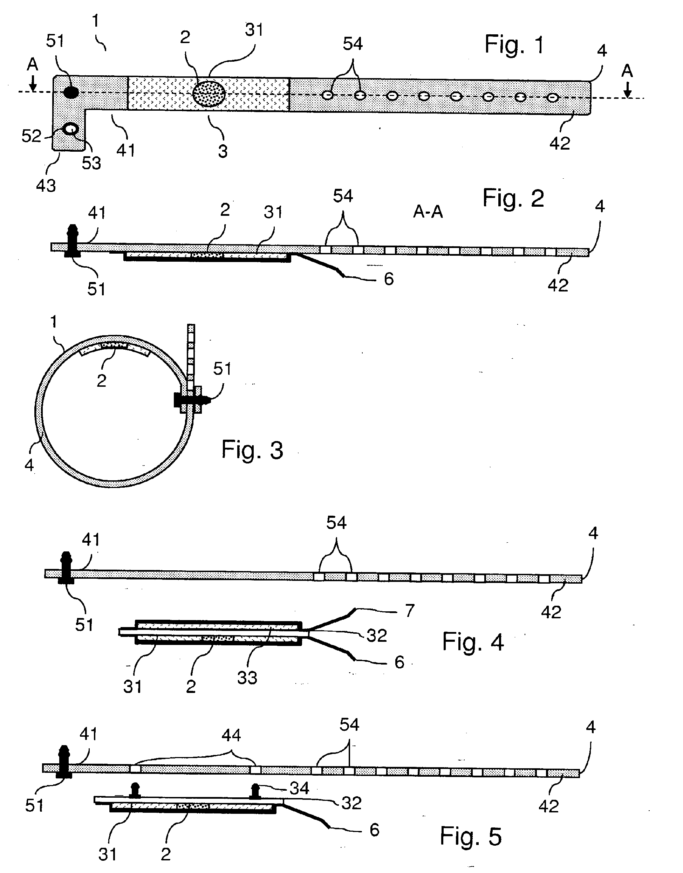

Les figures 1 à 3 illustrent plus précisément un premier mode de réalisation d'un dispositif 1 pour l'application cutanée d'une substance 2. Le dispositif 1 inclut un patch 3. Le patch 3 comprend la substance 2 et un adhésif 31, destinés à être appliqués sur la peau. L'adhésif 31 est disposé à proximité de la substance 2 afin d'améliorer son maintien en contact contre la peau du sujet. Avantageusement, l'adhésif 31 entoure la substance 2 pour garantir son contact optimal avec la peau, mais d'autres configurations sont bien entendu possibles (en bandes, spots, etc.). Le dispositif 1 comprend en outre un bracelet 4 à usage unique. Le bracelet 4 est muni d'un système de fixation irréversible permettant d'empêcher le retrait prématuré du dispositif, et ainsi de maintenir la substance 2 contre la peau du sujet pendant une période de temps suffisante.

Le bracelet 4 est formé sensiblement sous forme de bande. Le bracelet 4 comprend de façon connue en soi deux parties de fixation 41 et 42, disposées aux deux extrémités de la bande. De façon connue en soi, le bracelet 4 est flexible de sorte que les parties de fixation 41 et 42 puissent être jointes. Lorsque les parties de fixation 41 et 42 sont jointes, le bracelet 4 forme une boucle, comme illustré à la figure 3. Les moyens de fixation irréversibles comprennent des moyens pour fixer ensemble les parties 41 et 42 de façon irréversible. Ces moyens de fixation comprennent un organe de verrouillage disposé au niveau de la partie de fixation 41. L'organe de verrouillage comporte un plot 51 et un alésage 53. Le plot 51 et l'alésage 53 sont conformés pour que le plot 51 puisse être inséré de façon irréversible à travers l'alésage 53. Le plot 51 comprend ainsi une extrémité élargie, suivie d'une partie médiane plus mince. L'organe de verrouillage présente un anneau 52 délimitant l'alésage 53 et étant déformable élastiquement par le plot, de façon à permettre l'insertion irréversible du plot 51 à travers l'alésage 53. Une fois que l'extrémité élargie du plot 51 a traversé l'alésage 53, l'anneau se loge dans la partie médiane du plot 51.

L'anneau 52 est disposé sur une saillie latérale de la partie 41. Cette saillie latérale 43 est apte à être rabattue pour insérer le plot 51 dans l'alésage 53 de l'anneau 52. La partie de fixation 42 comprend plusieurs alésages 54 répartis dans le sens de la longueur du bracelet 4. Les différentes positions longitudinales des alésages permettent d'adapter la taille de la boucle formée à la dimension du membre du sujet. Comme illustré à la figure 3, la fixation des deux parties 41 et 42 est irréversible lorsque le plot 51 traverse à la fois un alésage 54 et l'alésage 53, la saillie latérale 43 étant rabattue sur le reste de la partie 42.

D'autres moyens de fixation irréversible des parties de fixation, connus de l'homme du métier dans le domaine des bracelets inviolables, pourront bien entendu être utilisés.

Comme illustré à la figure 2, le dispositif 1 comprend en outre un élément pelable de protection 6. L'élément pelable de protection 6 recouvre intégralement l'adhésif 31 et la substance 2, afin d'éviter leur détérioration avant leur application sur la peau. L'élément pelable 6 est muni d'une languette de préhension facilitant son pelage. Sur la figure 1, le dispositif est représenté en l'absence de l'élément pelable 6.

Dans ce mode de réalisation, le dispositif fourni comprend initialement un patch 3 solidaire du bracelet 4. Le bracelet 4 forme en pratique un support en contact avec l'adhésif 31 et la substance 2. L'irréversibilité de la fixation garantit donc l'application sur la peau de la substance 2 du patch 3 jusqu'à la rupture du bracelet 4 ou du système de fixation.

En remplacement ou en supplément de la fixation mécanique irréversible au moyens des éléments 51 à 54, on peut également envisager que les deux parties 41 et 42 présentent des surfaces mutuellement adhésives. L'homme du métier pourra déterminer un adhésif adéquat pour qu'une tentative d'ouverture de la boucle conduise à une détérioration visible du bracelet ou de l'adhésif.

Dans les modes de réalisation des figures 4 et 5, le patch 3 et le bracelet 4 sont fournis sous forme de kit. Le patch 3 et le bracelet 4 de ces modes de réalisation disposent de moyens de solidarisation mutuels. Ces moyens de solidarisation mutuels sont avantageusement irréversibles, de sorte que l'irréversibilité de la fixation garantit l'application sur la peau de la substance 2 du patch 3 jusqu'à la rupture du bracelet 4 ou du système de fixation. Ces modes de réalisation permettent par exemple de choisir un patch 3 présentant un dosage souhaité de la substance 2, puis de solidariser ce patch 3 de façon irréversible à un bracelet à usage unique 4. On pourra avantageusement prévoir que le bracelet 4 et le patch 3 soit disposés dans un même conditionnement non illustré.

Le dispositif 1 selon le mode de réalisation illustré à la figure 4 comprend un bracelet 4 similaire à celui du premier mode de réalisation. Le dispositif 1 comprend en outre un patch 3 destiné à être solidarisé irréversiblement au bracelet 4. Le patch 3 comprend un support 32, tel qu'un film synthétique. Une substance 2 et un adhésif 31 sont solidaires d'une première face du support 32. Un adhésif 33 est solidaire d'une deuxième face du support 32. L'adhésif 33 est destiné à solidariser le patch 3 de façon irréversible au bracelet 4. Le bracelet 4 dispose d'une surface destinée à assurer l'adhésion avec l'adhésif 33. Après la solidarisation du bracelet 4 et du patch 3, cette surface recouvre avantageusement intégralement la substance 2. Ainsi, on garantit le contact de la substance 2 avec la peau durant le port du bracelet 4.

Un élément pelable de protection 6 recouvre intégralement l'adhésif 31 et la substance 2. Un élément pelable de protection 7 recouvre intégralement l'adhésif 33, afin d'éviter sa détérioration avant son application contre le bracelet 4. Comme l'élément pelable 6, l'élément pelable 7 dispose d'un organe de préhension pour faciliter son pelage.

Le dispositif 1 selon le mode de réalisation illustré à la figure 5 comprend un bracelet 4 similaire à celui du premier mode de réalisation. Deux alésages 44 sont en outre ménagés dans une zone médiane du bracelet 4. Le dispositif 1 comprend en outre un patch 3 destiné à être solidarisé irréversiblement au bracelet 4. Le patch 3 comprend un support 32, tel qu'un film synthétique. Une substance 2 et un adhésif 31 sont solidaires d'une première face du support 32. Deux plots 34 sont en saillie depuis une deuxième face du support 32. Les plots 34 et les alésages 44 sont configurés pour garantir une solidarisation irréversible du bracelet 4 et du patch 3. Les alésages 44 peuvent pour cela être délimités par des anneaux similaires à l'anneau 52.

Dans les modes de réalisation des figures 4 et 5, on peut prévoir que le support 32 présente des propriétés électrostatiques sur la face en contact avec la substance 2. Un exemple de tel support est décrit par exemple dans la demande de brevet WO02/071950 ou WO2006/128981. On peut alors prévoir qu'une substance 2 sous forme de particules soit maintenue en contact avec le support 32 par des forces électrostatiques. Il est entendu toutefois que la présente demande peut être appliquée à tout type de patch.

## Revendications

1. Dispositif (1) pour l'application cutanée d'une substance (2) à un sujet, **caractérisé en ce qu'**il comprend :
- un patch (3) comprenant ladite substance (2); et
- un bracelet (4) à usage unique comprenant un système de fixation irréversible permettant de s'assurer qu'un temps de contact suffisant de la substance avec la peau a été respecté.

2. Dispositif (1) selon la revendication 1, dans lequel le bracelet comprend deux parties de fixation (41, 42) et dans lequel le système de fixation irréversible du bracelet comprend des moyens (51, 52, 53, 54) pour fixer ensemble de façon irréversible les deux parties de fixation, le bracelet (4) formant une boucle lorsque les deux parties de fixation sont jointes.

3. Dispositif (1) selon la revendication 2, dans lequel les deux parties de fixation comprennent des surfaces mutuellement adhésives.

4. Dispositif (1) selon la revendication 2, dans lequel :
- une des deux parties de fixation comprend un organe de verrouillage (51, 52, 53) présentant un alésage (53), et comprend un plot (51) apte à être inséré de façon irréversible à travers l'alésage ;
- l'autre des deux parties de fixation comprend au moins un alésage (54) susceptible d'être traversé par le plot.

5. Dispositif (1) selon la revendication 4, dans lequel l'organe de verrouillage présente un anneau (52) délimitant l'alésage (53) et déformable élastiquement de façon à permettre l'insertion irréversible du plot (51).

6. Dispositif (1) selon l'une quelconque des revendications précédentes, comprenant des moyens de solidarisation (33, 34) du patch au bracelet.

7. Dispositif (1) selon la revendication 6, dans lequel les moyens de solidarisation du patch au bracelet sont irréversibles.

8. Dispositif (1) selon la revendication 7, dans lequel les moyens de solidarisation irréversible comprennent une surface du patch ou du bracelet enduite d'adhésif (33) et recouverte d'un élément pelable de protection (7).

9. Dispositif (1) selon la revendication 7 ou 8, dans lequel les moyens de solidarisation irréversible du patch (3) au bracelet (4) comprennent un organe (34) du patch et un organe (44) du bracelet mutuellement encliquetables.

10. Dispositif (1) selon l'une quelconque des revendications 7 à 9, comprenant un conditionnement contenant le bracelet (4) et le patch (3).

11. Dispositif (1) selon l'une quelconques des revendications 1 à 6, dans lequel le bracelet (4) présente une surface en contact avec la substance (2).

12. Dispositif (1) selon la revendication 11, dans lequel le bracelet (4) présente une surface adhésive (31) à proximité de la substance (2).

13. Dispositif (1) selon la revendication 12, dans lequel la surface adhésive (31) entoure la substance (2).

14. Dispositif (1) selon l'une quelconque des revendications 1 à 10, dans lequel le patch comprend un support (32) muni d'une face présentant une surface en contact avec la substance (2) et une surface adhésive (31) destinée à entrer en contact avec la peau.

15. Dispositif (1) selon l'une quelconque des revendications précédentes, dans lequel le patch (3) comprend un élément de protection pelable (6) solidaire, le cas échéant, du support (32) ou du bracelet (4) et recouvrant la substance (2).

16. Dispositif (1) selon l'une quelconque des revendications précédentes, dans lequel le bracelet (4) recouvre la substance (2).

## Patentansprüche

1. Vorrichtung (1) für die kutane Applikation einer Substanz (2) bei einer Person, **dadurch gekennzeichnet, dass** sie umfasst:
- ein Pflaster (3), umfassend besagte Substanz (2); und
- ein Armband (4) zum einmaligen Gebrauch, umfassend ein irreversibles Verbindungssystem, das die Einhaltung einer hinreichenden Kontaktzeit der Substanz mit der Haut sicherstellen kann.

2. Vorrichtung (1) gemäß Anspruch 1, wobei das Armband zwei Verbindungsteile (41, 42) umfasst und wobei das irreversible Verbindungssystem des Armbands Mittel (51, 52, 53, 54) umfasst, um die beiden Verbindungsteile miteinander irreversibel zu verbinden, wobei das Armband (4) eine Schleife bildet, wenn die beiden Verbindungsteile miteinander verknüpft sind.

3. Vorrichtung (1) gemäß Anspruch 2, wobei die beiden Verbindungsteile gegenseitige Klebeflächen umfassen.

4. Vorrichtung (1) gemäß Anspruch 2, wobei:
- eines der beiden Verbindungsteile ein Verschlusselement (51, 52, 53) umfasst, das eine Bohrung (53) aufweist und einen Pin (51) umfasst, der irreversibel durch die Bohrung hindurch inseriert werden kann;
- das andere der beiden Verbindungsteile wenigstens eine Bohrung (54) umfasst, durch die der Pin geführt werden kann.

5. Vorrichtung (1) gemäß Anspruch 4, wobei das Verschlusselement einen Ring (52) aufweist, der die Bohrung (53) begrenzt und elastisch verformbar ist, um die irreversible Insertion des Pins (51) zu ermöglichen.

6. Vorrichtung (1) gemäß einem der vorhergehenden Ansprüche, umfassend Mittel zur Befestigung (33, 34) des Pflasters am Armband.

7. Vorrichtung (1) gemäß Anspruch 6, wobei die Mittel zur Befestigung des Pflasters am Armband irreversibel sind.

8. Vorrichtung (1) gemäß Anspruch 7, wobei die Mittel zur irreversiblen Befestigung eine Oberfläche des Pflasters oder des Armbands umfassen, die mit Klebstoff (33) beschichtet ist und mit einem abziehbaren Schutzelement (7) abgedeckt ist.

9. Vorrichtung (1) gemäß Anspruch 7 oder 8, wobei die Mittel zur irreversiblen Befestigung des Pflasters (3) am Armband (4) ein Element (34) des Pflasters und ein Element (44) des Armbands umfassen, die gegenseitig einrastbar sind.

10. Vorrichtung (1) gemäß einem der Ansprüche 7 bis 9, umfassend eine Verpackung, die das Armband (4) und das Pflaster (3) enthält.

11. Vorrichtung (1) gemäß einem der Ansprüche 1 bis 6, wobei das Armband (4) eine Oberfläche aufweist, die mit der Substanz (2) in Kontakt kommt.

12. Vorrichtung (1) gemäß Anspruch 11, wobei das Armband (4) eine Klebefläche (31) in der Nähe der Substanz (2) aufweist.

13. Vorrichtung (1) gemäß Anspruch 12, wobei die Klebefläche (31) die Substanz (2) umgibt.

14. Vorrichtung (1) gemäß einem der Ansprüche 1 bis 10, wobei das Pflaster einen Träger (32) umfasst, dessen eine Seite eine Oberfläche, die mit der Substanz (2) in Kontakt kommt, und eine Klebefläche (31), die mit der Haut in Kontakt treten soll, aufweist.

15. Vorrichtung (1) gemäß einem der vorhergehenden Ansprüche, wobei das Pflaster (3) ein abziehbares Schutzelement (6) umfasst, das gegebenenfalls mit dem Träger (32) oder mit dem Armband (4) verbunden ist und die Substanz (2) abdeckt.

16. Vorrichtung (1) gemäß einem der vorhergehenden Ansprüche, wobei das Armband (4) die Substanz (2) abdeckt.

## Claims

1. A device (1) for cutaneously applying a substance (2) to a subject, **characterized in that** it comprises:
- a patch (3) comprising said substance (2); and
- a single-use bracelet (4) comprising an irreversible attachment system allowing to ensure that a sufficient contact time of the substance with the skin has been observed.

2. The device (1) according to claim 1, wherein the bracelet comprises two attachment portions (41, 42) and wherein the irreversible attachment system of the bracelet comprises means (51, 52, 53, 54) for irreversibly attaching together both attachment portions, the bracelet (4) forming a loop when both attachment portions are joined together.

3. The device (1) according to claim 2, wherein both attachment portions comprise mutually adhesive surfaces.

4. The device (1) according to claim 2, wherein:
- one of the two attachment portions comprises a locking member (51, 52, 53) having a bore (53), and comprises a stud (51) capable of being irreversibly inserted through the bore;
- the other of the two attachment portions comprises at least one bore (54) capable of being crossed by the stud.

5. The device (1) according to claim 4, wherein the locking member has a ring (52) delimiting the bore (53) and elastically deformable so as to allow irreversible insertion of the stud (51).

6. The device (1) according to any of the preceding claims, comprising means (33, 34) for securing the patch to the bracelet.

7. The device (1) according to claim 6, wherein the means for securing the patch to the bracelet are irreversible.

8. The device (1) according to claim 7, wherein the irreversible securing means comprise a surface of the patch or of the bracelet coated with an adhesive (33) and covered with a peelable protective element (7).

9. The device (1) according to claim 7 or 8, wherein the means for irreversibly securing the patch (3) to the bracelet (4) comprise a member (34) of the patch and a member (44) of the bracelet which may be snapped on mutually.

10. The device (1) according to any of claims 7 to 9, comprising a packaging containing the bracelet (4) and the patch (3).

11. The device (1) according to any of claims 1 to 6, wherein the bracelet (4) has a surface in contact with the substance (2).

12. The device (1) according to claim 11, wherein the bracelet (4) has an adhesive surface (31) in proximity to the substance (2).

13. The device (1) according to claim 12, wherein the adhesive surface (31) surrounds the substance (2).

14. The device (1) according to any of claims 1 to 10, wherein the patch comprises a support (32) provided with a face having a surface in contact with the substance (2) and an adhesive surface (31) intended to enter into contact with the skin.

15. The device (1) according to any of the preceding claims, wherein the patch (3) comprises a peelable protective element (6) secured when present to the support (32) or to the bracelet (4) and covering the substance (2).

16. The device (1) according to any of the preceding claims, wherein the bracelet (4) covers the substance (2).
